# EUROPEAN PATENT SPECIFICATION

(11) **EP 1 372 476 B1**
(45) Date of publication and mention of the grant of the patent: **21.05.2008**
(21) Application number: 02705017.8
(22) Date of filing: 19.03.2002
(51) Int. Cl.: A61B 5/055, G01R 33/28

(54) **CATHETER FOR USE IN A MAGNETIC RESONANCE IMAGING APPARATUS**
KATHETER VERWENDET IN EINEM MAGNET RESONANZ ABBILDUNGSGERÄT
CATHETER UTILISABLE DANS UN APPAREIL D'IMAGERIE A RESONANCE MAGNETIQUE

(30) Priority: 21.03.2001 DE 10113661
(43) Date of publication of application: 02.01.2004
(73) Proprietor: Koninklijke Philips Electronics N.V., 5621 BA Eindhoven (NL); Philips Intellectual Property & Standards GmbH, 20099 Hamburg (DE)
(72) Inventor: LEUSSLER, Christoph, G., NL-5656 AA Eindhoven (NL)
(74) Representative: Volmer, Georg
(86) International application number: PCT/IB2002/000876
(87) International publication number: WO 2002/074164

(56) References cited:
- WO-A-00/48512
- US-A- 4 572 198
- US-A- 5 050 607
- US-A- 5 353 795

## Description

The invention relates to a catheter for use during examination of an object by means of a magnetic resonance (MR) imaging apparatus. The invention also relates to a method of forming an MR image and of visualizing a position of a catheter, introduced into an object to be examined, in the MR image, as well as to an MR imaging apparatus for carrying out such a method.

As is known, magnetic resonance imaging apparatus are used not only for the examination but also for the treatment of a patient. In many cases a catheter is then introduced into the patient, and it must be possible to localize the catheter in the MR image. However, because the catheter is generally made of materials which are not visible in an MR image, steps must be taken so as to ensure that notably the tip of the catheter can be reliably recognized without affecting the patient.

In principle a distinction is made between passive and active methods for this purpose. In the case of a passive method, for example, as known from WO 99/19739, one or more small-resonant circuits at the tip of the catheter become visible in the MR image in that they cause an increase of the RF field (B₁ field) in their immediate vicinity and hence increase the magnetization of the neighboring nuclear spins. The increase of the RF field is directly proportional to the quality factor of the resonant circuit. However, because this quality factor generally is very small, the resonant circuit must be switched on and off so as to enable its position, and hence the position of the catheter tip, to be determined by way of a subtraction method. Active switches (for example, diodes) which are switched in the resonant circuit for this purpose degrade the quality factor of the resonant circuit further, so that the magnetization (flip angle of the nuclear spins) is changed to a very small degree only, that is, notably in the case of complex body structures. Furthermore, the quality factor of the resonant circuit is particularly poor in the case of low field strengths, because the coil losses are then proportionally higher.

EP 0 531 081 discloses an active system in which the catheter is provided with a transmission coil which is connected to an RF source and is fed with a comparatively low power, so that the coil generates an electromagnetic dipole field which is picked up by external receiving coils and evaluated so as to determine or track the position of the catheter. This active system, however, has a drawback in that, in addition to the catheter, wires which carry RF voltages must be introduced into the patient so as to supply the transmission coil with the RF power. This may give rise to undesirable heating of the tissue in the vicinity of the wires, so that operation can take place with a comparatively small power only. Moreover, the higher the frequency of the RF power, the higher the losses will be, so that this principle can be used only in the range of comparatively low frequencies. A low power in conjunction with low frequencies, however, may significantly affect the possibilities for localizing the catheter. Furthermore, the amount of calculation work necessary for determining a suitably accurate position is comparatively large. Finally, the RF power emitted by the wires could cause disturbances in the MR image.

Therefore, it is an object of the present invention to provide a catheter which is intended notably for use during the examination of a patient or another object by means of an MR imaging apparatus and can be visualized significantly better in an MR image while using comparatively few additional means. In accordance with the invention this is object is achieved by a catheter as defined in claim 1.

Furthermore, it is an object to provide a method of forming an MR image and of simply and clearly visualizing in the MR image a position of a catheter introduced into an object to be examined. This object is achieved by a first method as defined in claim 7 or by a second method as defined in claim 8. Whereas the first method is generally to be preferred, for given applications it may also be useful to operate the RF coil and the transmission unit of the catheter simultaneously in conformity with the second method, for example, in order to carry out specific examinations during localization of the (moving) catheter. A special advantage of the first method resides in the fact that, because of the alternating modes of operation, mutual influencing or disturbing of the imaging on the one hand and the localizing of the catheter on the other hand is practically precluded.

In the context of the this invention the near field mentioned in the above claims is to be understood to mean the zone which is enclosed by the transmission antenna as well as the zone outside the transmission antenna in which the RF field of the antenna is essentially concentrated.

The foregoing solutions combine the advantages of passive and active systems, without having to accept the essential drawbacks thereof. On the one hand, the accuracy of localization is exactly as high as in the case of the described passive system, but the local nuclear magnetization, and hence also the MR relaxation signal to be acquired, is essentially higher because of the active excitation. On the other hand, the means required for evaluating the signal received are significantly less than in the case of the described active system, because the signal is picked up by the RF receiving coils which are present in an MR imaging apparatus any way and can be processed and reproduced by the devices present therein,

The dependent claims relate to further advantageous embodiments of the invention.

The embodiment disclosed in the claims 2 and 5 offer special advantages in respect of ease of manufacture of a catheter in accordance with the invention. The embodiment in conformity with claim 3 enables measurements to be performed at the same time in the tissue of a patient which surrounds the catheter, the measuring values being transferred preferably by means of a method as claimed in claim 9. The embodiments disclosed in the claims 4 and 6 are particularly advantageous with a view to the use of the catheter without complications, whereas a method as claimed in claim 10 even enables the supply lead through the catheter to be dispensed with. Finally, the claims 11 and 12 describe a magnetic resonance imaging apparatus which is particularly suitable for carrying out the method.

Further details, features and advantages of the invention will become apparent from the following description of preferred embodiments which is given with reference to the drawing. Therein:
Fig. 1 is a diagrammatic side elevation of an MR imaging apparatus;
Fig. 2 shows a catheter in accordance with the invention,
Fig. 3 shows a first block diagram of a transmission unit,
Fig. 4 shows a second block diagram of a part of the transmission unit,
Fig. 5 shows equivalent diagrams of an antenna of the transmission unit,
Fig. 6 illustrates the principle of a microchip with a transmission unit,
Fig. 7 shows various embodiments of the catheter,
Fig. 8 shows a preferred embodiment of the catheter,
Fig. 9 shows a first embodiment of the tip of a catheter,
Fig. 10 shows a second embodiment of the tip of a catheter,
Fig. 11 shows further alternative embodiments of the tip of a catheter,
Fig. 12 shows frequency spectra of the signals emitted by the transmission unit,
Fig. 13 is a side elevation of an array comprising a plurality of receiving antennas, and
Fig. 14 is a three-dimensional view of the arrangement of receiving antennas.

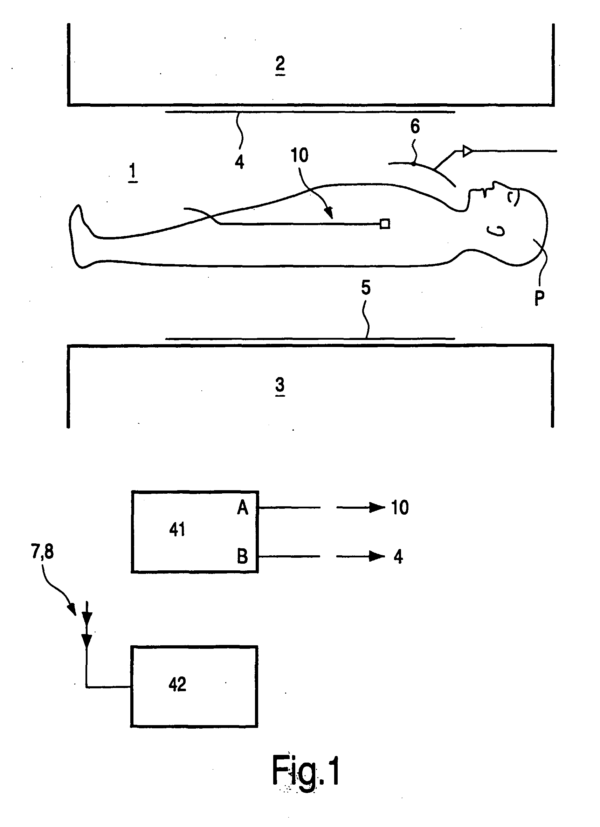

Fig. 1 shows the essential components of an MR imaging apparatus which relate to the generating and acquisition of magnetic fields in an examination zone 1. Above and below the examination zone 1 there are provided respective magnet systems 2, 3 which serve to generate a basic magnetic field (B₀ field) as well as gradient magnetic fields in known manner. The basic magnetic field traverses a patient P essentially in a direction perpendicular to the longitudinal axis of the patient (that is, generally in the vertical direction).

Flat, or at least flattish, RF conductor structures (flat resonators) in the form of RF transmission coils 4 and RF receiving coils 5 serve to generate RF pulses (B₁ field) whereby the nuclear spins are excited in the tissue to be examined as well for the detection of subsequent MR relaxation processes in the tissue, said RF transmission and receiving coils being arranged on the magnet systems 2 and 3, respectively; additionally, or alternatively, a local RF receiving coil 6 may also enclose the part of the patient P to be examined.

As an alternative for this so-called vertical system, the catheter in accordance with the invention can also be used in axial systems in which the basic magnetic field traverses the patient essentially in the direction of the longitudinal axis of the patient. Such systems are known per se and comprise a tubular examination space in which the patient is introduced in the axial direction.

A catheter 10 is often use to perform a treatment on the patient P or to take a tissue sample, the catheter being introduced into the patient and its position being visualized on a display screen.

To this end, there is provided a switching unit 41 which is connected to the catheter 10 by way of a first output A and to the RF transmission coils 4 by way of a second output B. The catheter 10 and the RF transmission coils 4 can be manually or automatically controlled by means of the switching unit in such a manner that they are alternately or essentially simultaneously operative in the manner to be described hereinafter.

In the alternating mode (first method), switching over takes place between two modes of operation. In the first mode of operation, the RF transmission coil 4 of the MR imaging apparatus is operative in known manner so as to form an MR image of the object to be examined; and the transmission unit 11 of the catheter 10 is switched off.

In the second mode of operation, on the one hand the RF transmission coil 4 is switched off. On the other hand, an activated transmission unit arranged at the tip of the catheter excites a local nuclear magnetization (that is, in the near field of a transmission antenna of the catheter) by emission of RF pulses of a first frequency f₁, and the resultant MR relaxation events are received by the RF receiving coils 5, 6. The signal received serves to reproduce the position of the catheter tip in the MR image.

In the simultaneous mode of operation (second method), the RF transmission coils of the MR imaging apparatus and the transmission unit of the catheter are activated essentially simultaneously, so that an intensified nuclear magnetization is excited in the near field of the transmission antenna; such intensified nuclear magnetization is detected by the RF receiving coils 5, 6 and is reproduced in the MR image formed so as to visualize the position of the catheter.

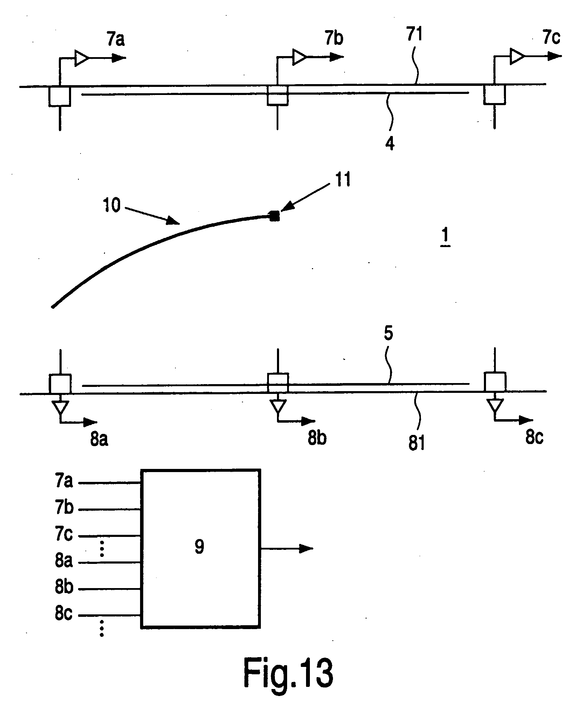

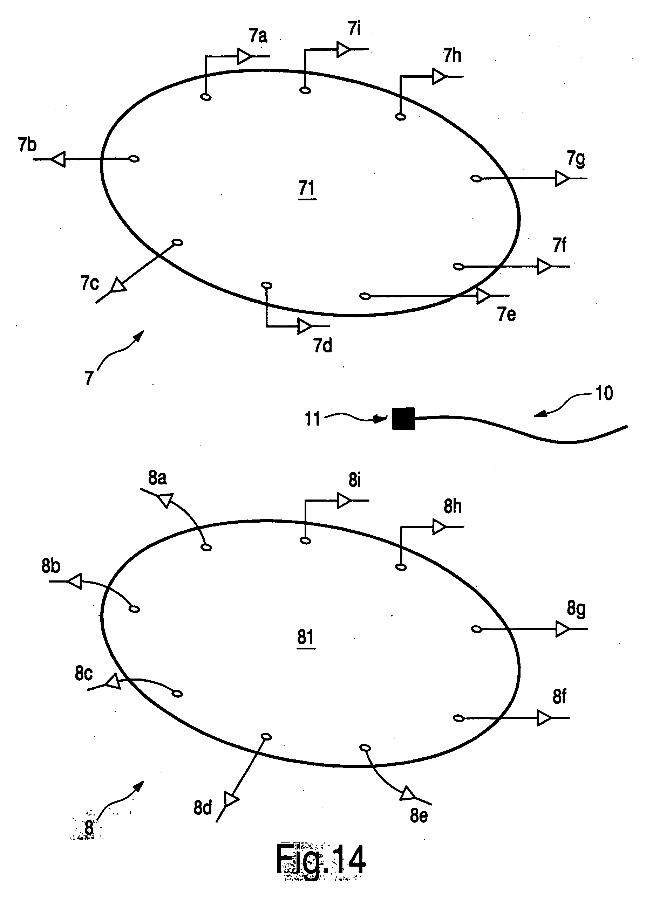

The transmission unit may also include sensors for picking up given properties of the surrounding tissue. In that case the measuring values of the sensors are applied, by way of modulation and emission of electromagnetic waves of a second frequency f₂ (or of acoustic or optical waves), to a receiving and evaluation unit 42 which includes a receiving antenna array 7x, 8x (see Figs. 13 and 14) so as to be further processed or displayed in known manner.

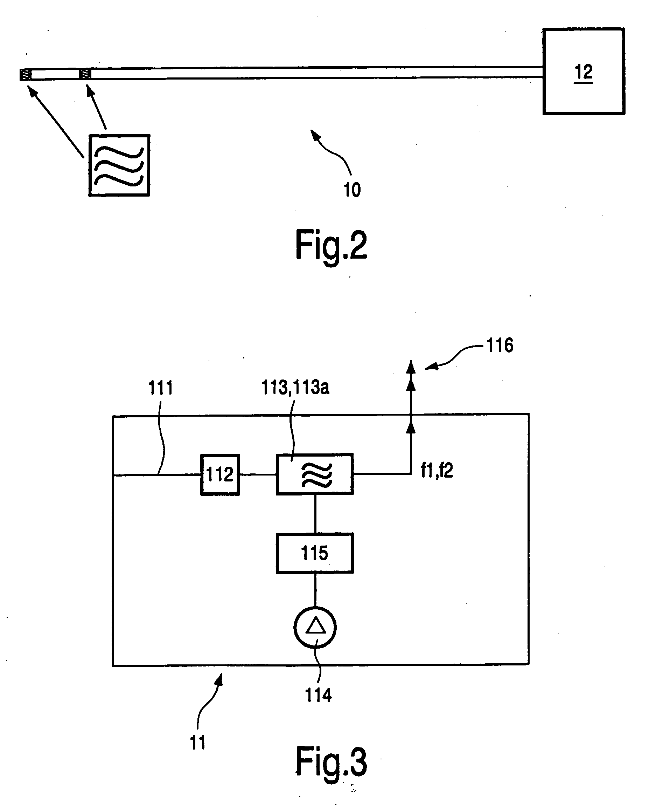

Fig. 2 is a diagrammatic representation of a catheter 10 in accordance with the invention. At the tip of the catheter, or in a position which is situated at a small distance therefrom, there is provided a microchip 11 (additionally shown at an enlarged scale) which includes the transmission unit. The microchip includes all components necessary for generating RF electromagnetic waves. At the other end of the catheter 10, that is, the end situated outside the patient, there is provided a supply and control unit 12 via which a supply and control lead which is guided through the catheter is connected to the microchip 11.

Fig. 3 shows a block diagram of the microchip 11. The microchip includes an energy converter 112 which is connected to the supply and control lead 111 and supplies a preferably voltage-controlled RF oscillator 113 (VCO) with a suitable supply voltage. The RF oscillator 113 is also controlled via the lead 111 and includes a switch-off unit 113a. An output of the RF oscillator is connected to at least one transmission antenna 116 for the emission of the generated electromagnetic waves of a first frequency f₁. The first frequency f₁ serves to generate a local B₁ field whereby a nuclear magnetization is excited in the tissue in the near field of the transmission antenna 116.

Also provided on the microchip 11 are one or more sensors 114 for picking up temperatures, pressures, pH-values or other parameters of the surrounding tissue. The output signals of the sensors are applied to a modulator 115 for modulating the frequency, amplitude and/or phase of the electromagnetic waves of a second frequency f₂ which are generated by the RF oscillator 113, so that the sensor signals can be transmitted in a wireless manner, via the transmission antenna 116, to the receiving and evaluation unit 42, situated outside the patient, so as to be evaluated for diagnostic purposes.

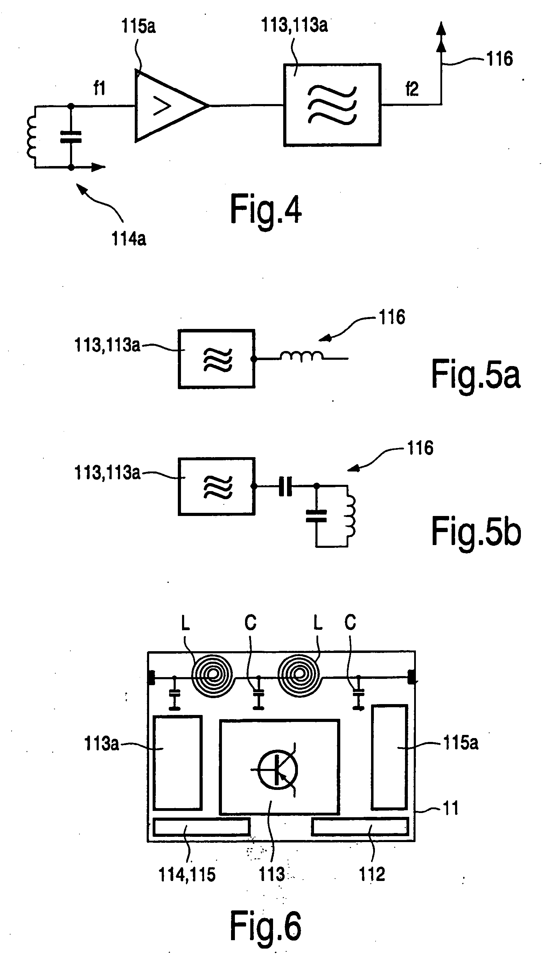

As is shown in Fig. 4, a sensor may also be a resonant circuit 114a or an inductance such as, for example, a separate MR receiving coil whereby the MR relaxation process or processes in the near field of the sensor subsequent to the excitation with the first frequency f₁ are picked up. This sensor 114a is connected to the input of a preamplifier 115a whose output is connected to the oscillator 113, so that the excited MR relaxation events can also be emitted, via the transmission antenna 116, in the form of a suitable modulation of the second frequency f₂, that is, if they are not picked up by the RF receiving coils 5, 6. In this case the catheter should be localized by direction finding of the receiving direction in different projections; preferably a plurality of suitably arranged receiving antennas is used for this purpose.

When the first frequency and the second frequency are close enough to one another, a common transmission antenna 116 can be used for the two frequencies. When separate transmission antennas are provided, the transmission antenna for the first frequency can also serve as the sensor 114a for picking up the MR relaxation events.

The oscillator may be constructed, for example as a Colpits oscillator or a Hartley oscillator and includes one or more resonant circuits as well as a switch-off unit 113a (essentially a diode) for switching off the resonant circuits so as to avoid induction phenomena when the RF transmission coils 4 emit RF pulses (generating the B₁ field) in order to excite the nuclear spins in the object to be examined.

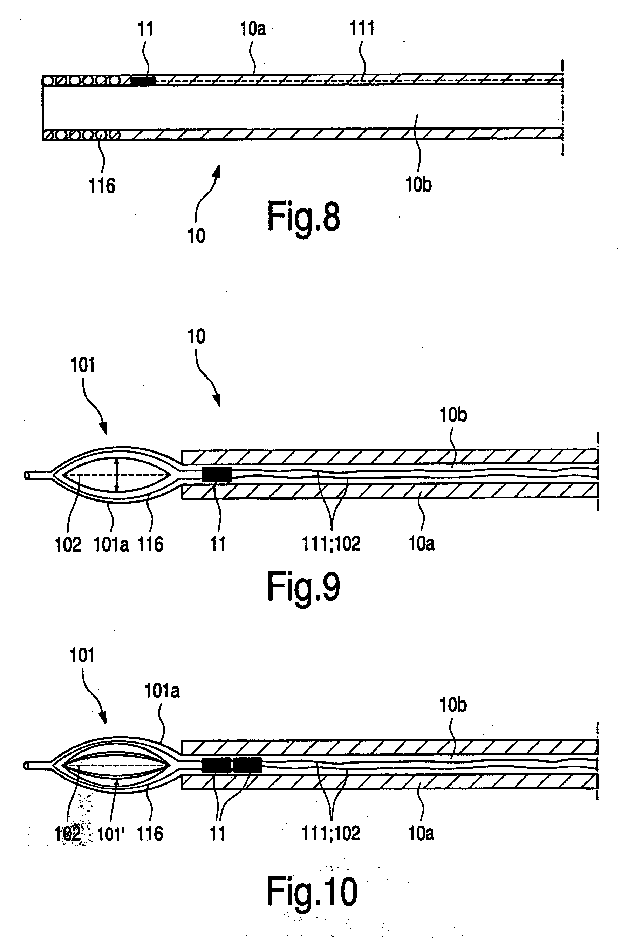

The transmission antenna 116 may be formed by one or more microcoils which form, in conformity with Fig. 5, either a short rod antenna (a) or are connected, for example, so as to form a parallel resonant circuit (b) which is capacitively coupled to the oscillator 113. These coils are preferably wound directly around the tip of the catheter, that is, separate from the microchip (Figs. 8 to 11).

The microchip 11 is formed, for example, as a silicon chip in a bipolar technique or on the basis of a glass or a ceramic material. A chip of this kind, occupying a surface area of no more than from approximately 1 to 4 mm², can be integrated directly in the tip of the catheter and is diagrammatically shown in a plan view in Fig. 6.

Various assemblies which form respective electronic functional units are indicated on the chip. These functional units include the oscillator 113 which receives electric power via the energy converter 112, a sensor and modulator unit 114, 115 which includes one or more sensors with associated measuring data modulators, the preamplifier 115a as well as the switch-off unit 113a for the oscillator 113 or the transmission antenna 116. Furthermore, one or more inductances (planar, coils) L and capacitances C, constituting one or more resonant circuits of the oscillator 113, are also printed as appropriate conductor tracks on the chip 11.

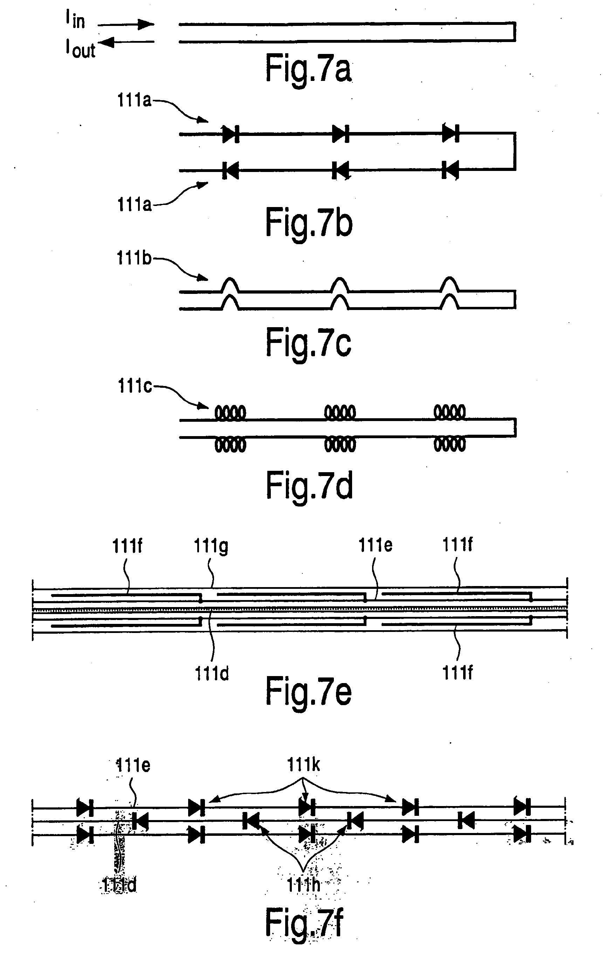

The power supply and control of the microchip 11 take place via the supply and control lead 111, various embodiments of which are shown in Fig. 7.

In the simplest case, this lead is formed by a thin wire as shown in Fig. 7(a). In order to avoid a temperature increase due to resonance effects, the supply lead 111 is preferably made of resistance wire alloys having a resistance of more than 500 ohms/m. Alternatively, the supply lead may also be formed as an alternating sequence of short copper wire leads and resistance wire leads of a length 1 of each time 1 << λ/4 (λ = wavelength of the emitted energy) in order to reduce the low-frequency overall resistance and avoid standing waves. Furthermore, a parallel connection of a plurality of very thin supply wires is also possible. In most cases it suffices to provide only one conductor (forward conductor) and to use the capacitance of the body of the patient as the return conductor.

A plurality of diodes 111a may be connected in series in the supply lead as shown in Fig. 7(b) so as to avoid that it tends to resonate. In order to preclude an excessive current through the supply lead in all cases, in conformity with Fig. 7(c) a plurality of serial fuses 111b is provided so as to interrupt the lead when a limit current is exceeded. In order to suppress RF currents, in conformity with Fig. 7(d) a plurality of inductances 111c may also be connected in series in the supply lead.

Fig. 7(e) shows a further embodiment of the supply lead 111. This lead forms a coaxial cable which comprises an inner conductor 111d enclosed by an outer conductor 111e which is formed as a shield. Furthermore, a plurality of λ/4 leads 111f is arranged along the coaxial cable, one end of said λ/4 leads being connected to the outer conductor 111e whereas their other end is open. Finally, the coaxial cable and the λ/4 leads preferably are also enclosed by an insulating sheath 111g. The propagation of RF currents via the supply lead, and hence the risk of resonances, is avoided again in this embodiment.

Finally, Fig. 7(f) shows a further version of the supply lead shown in Fig. 7(b). It is a coaxial lead in the inner conductor 111d of which a first plurality of diodes 111h is connected in series in a first current direction, whereas a second plurality of diodes 111k which are connected in series in a second, opposite current direction is inserted in the outer conductor 111e. The first diodes 111h and the second diodes 111k are arranged so as to be offset relative to one another.

The supply lead 111 may also be an optical fiber. In that case the energy converter 112 is an optoelectronic converter which forms the necessary supply voltage from the light.

In all of the above cases the supply lead 111 is suitable not only for the transfer of energy, but also for the control of the microchip and notably of the RF oscillator (VCO) so as to switch over between the first and the second frequency.

Because the RF transmission coil 4 and the transmission antenna 116 generally are operative in an alternating fashion, the microchip 11 can also be inductively supplied with the necessary power. To this end, for example, the field of the RF transmission coil 4 can be used to induce a voltage in the transmission antenna 116 (or another inductance), said voltage being capacitively stored and used to operate the microchip. In that case the supply lead 111 serves only for the control of the VCO 113. Even though the transmission power of the oscillator 113 is then smaller, of course, it generally suffices at least in the cases where use is made of an RF receiving coil 6 which is arranged directly around the part of the patient to be examined.

Feasible embodiments of the tip of the catheter will be described in detail hereinafter with reference to the Figs. 8 to 11.

Fig. 8 is a diagrammatic cross-sectional view of the front part of a catheter 10 and its tip. A catheter sleeve 10a, enclosing a catheter lumen 10b, accommodates the supply lead 111 (denoted by dashed lines). The lead terminates at the microchip 11 which is also accommodated in the catheter sleeve 10a. Finally, at the area of the front end of the catheter a microcoil (NMR coil) which serves as a transmission antenna 116 and whose turns are also situated in the catheter sleeve 10a is wound around the catheter.

The tip of the catheter may also be constructed as an expansion catheter 101 as shown in Fig. 9. In this case the turns of the coil 116 are provided in a wall 101a of the expansion catheter which is in known manner slid out of the catheter sleeve 10a so as to be expanded subsequently by means of a pulling wire 102 which extends through the catheter as far as its other end, or to be expanded automatically. The microchip 11 which is supplied with electric power and is controlled via the supply lead 111, in this case extending through the catheter lumen 10b, is again diagrammatically indicated in this representation.

Fig. 10 shows a further embodiment of the tip of the catheter which is formed by two orthogonal expansion catheters 101, 101', in the walls 101a of which there are again provided turns of one or more microcoils 116. The expansion catheters 101, 101' may also be expanded by way of the pulling wire 102 or automatically when slid out of the catheter sleeve 10a. The expansion catheter can be formed in such a manner that it is at the same time rotated about its longitudinal axis when the pulling wire 102 is pulled, so that it assumes a helix-like shape. The effect of the microcoils 116 can thus be optimized. Finally, the microchip 11 as well as the supply lead 111 and the pulling wire 102 which extend through the lumen 10b of the catheter are diagrammatically indicated again in this representation.

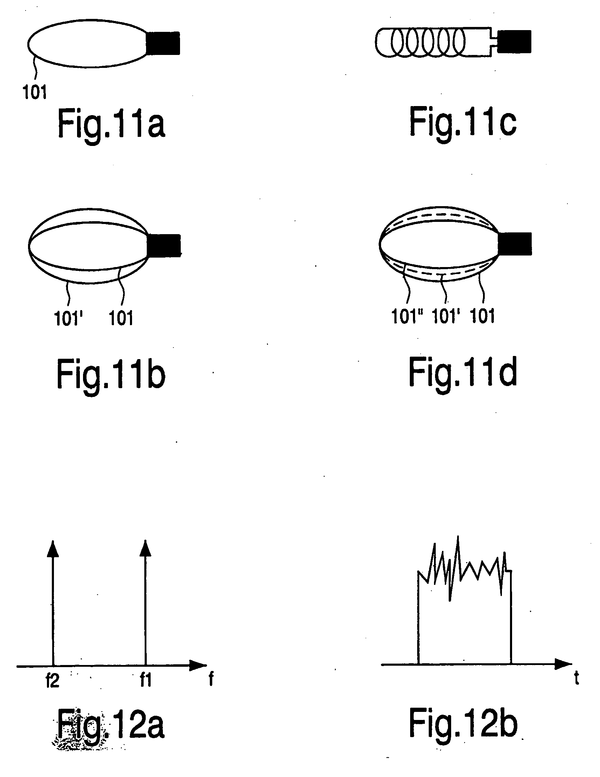

Fig. 11 shows this expansion catheter and other expansion catheters once more in separate views. In addition to the simple loop 101 (Fig. 11a) in conformity with Fig. 9 as well as the two orthogonal loops 101, 101' (Fig. 11b) in conformity with Fig. 10, a coil-like expansion catheter (Fig. 11c) as well as a multi-loop expansion catheter (Fig. 11d) can be used. The type of expansion catheter is chosen not only in dependence on the organ to be treated, but also with a view to achieving an optimum effect, that is, optimum nuclear magnetization, of the microcoils 116 formed by the individual loops.

The operation of the catheter in accordance with the invention will be described in detail hereinafter with reference to the Figs. 12 to 14.

Fig. 12(a) shows the relative position of the two frequencies produced by the RF oscillator 113, that is, the first frequency f₁ (NMR frequency) of RF pulses for generating the B₁ field whereby nuclear magnetization is excited in a near field around the transmission antenna (or the microcoil (coils)) 116, as well as the second frequency f₂ of an RF carrier whereby the measuring values and data picked up by the sensors 114, 114a are transferred to the receiving and evaluation unit 42 by frequency, amplitude or phase modulation. The first frequency f₁ is dependent on the strength of the basic magnetic field and lies between approximately 8 and 128 MHz for a range of the field strength of between 0.2 and 3 Tesla.

The second frequency f₂ may also be higher than the first frequency f₁, and each frequency may also be associated with a respective transmission antenna 116 and be connected to the oscillator output accordingly.

Furthermore, Fig. 12(b) also shows a typical short burst pulse with which the oscillator 113 emits electromagnetic waves of the first frequency f₁. The phase, the amplitude and the on/off times of the RF pulse as well as the bandwidth of the burst pulse can be varied so as to excite, by way of a corresponding adjustment of these variables, a nuclear magnetization to a different degree (for example, in dependence on the location in which the catheter is situated within the patient) or in an optimized manner.

During the examination of a patient by means of an MR imaging apparatus utilizing a catheter in accordance with the invention, switching over between two modes of operation preferably takes place in an alternating fashion.

In the first mode of operation the MR imaging apparatus is operative so as to form an MR image of the region to be examined in known manner, that is, the nuclear spins aligned by the basic magnetic field (B₀ field) are deflected, by the RF pulses (B₁ field) generated by the RF transmission coils 4, through a so-called flip angle (excitation by nuclear magnetization) and the subsequent relaxation events are detected by the RF receiving coils 5, 6 and localized so as to perform imaging by means of the gradient magnetic fields. The resonant circuit L, C of the oscillator 113 as well as preferably the transmission antenna 116 are then interrupted or separated from the oscillator by the switch-off unit 113, so that no currents can be induced therein. Furthermore, in this first mode of operation the voltage induced in the transmission antenna 116 of the microchip 11 (or in another inductance) can be capacitively stored.

In the second mode of operation the catheter 10 introduced into the patient is localized and reproduced in the MR image. In this mode of operation the RF transmission coils 4 are inactive and the VCO oscillator 113 is controlled in such a manner that the emission of electromagnetic waves of the first frequency f₁, that is, in the form of burst pulses (B₁ field), excites nuclear magnetization in the near field of the transmission antenna 116 (for example, a microcoil). The subsequent MR relaxation events are picked up again by the RF receiving coils 5, 6, localized by means of the gradient magnetic fields and reproduced in the MR image formed in the first mode of operation. In order to optimize the visibility, the amplitude, the frequency and/or the phase of the burst pulses preferably are adjustable.

Furthermore, in this second mode of operation the second frequency f₂ is amplitude modulated, frequency modulated and/or phase modulated, that is, by means of the measuring data modulator 115, with the measuring data picked up by the sensors 114, 114a so as to be emitted. Switching over between the first frequency and the second frequency preferably takes place in an alternating fashion by appropriate control of the (voltage-controlled) RF oscillator 113 via the supply lead 111.

For the reception of the measuring data emitted with the second frequency f₂ by the transmission unit 11 of the catheter 10, a plurality of local receiving antennas 7x, 8x is provided in the case of a vertical system as shown in the Figs. 13 and 14, each of said antennas being arranged at the periphery of a first planar RF shield 71 and a second planar RF shield 81, thus constituting a first antenna array 7 and a second antenna array 8. The RF shields 71, 81 are situated above and below the patient P in the examination space 1, that is, essentially at the area where the RF transmission coil 4 and the receiving coil 5 are arranged. Consequently, only a small transmission power is required for the oscillator 113, so that the current through the supply lead 111 is small and hence inductive voltage supply by the RF pulses generated during the first mode of operation possibly suffices. Fig. 13 is a longitudinal sectional view of the tubular examination space in the case of an axial system of the kind set forth.

Each of the receiving antennas 7x, 8x is connected to a diversity switch 9 whereby switching over to the receiving antenna which is each time nearest to the catheter takes place. The demodulation and the evaluation of the received measuring data take place by means of the receiving and evaluation unit 42 (Fig. 1) and while utilizing a method that is known per se.

Alternatively, it would also be possible, that is, if the transmission power is sufficiently high, to evaluate the amplitudes and the phases of all antenna signals 7x, 8x and to calculate the position of the tip of the catheter (that is, of the transmission antenna 116) relative to the first and the second antenna array 7, 8 in known manner on the basis of the amplitude differences and the phase differences.

## Claims

1. A catheter for use during examination of an object by means of an MR imaging apparatus with an RF transmission coil (4) wherein on the catheter (10) there are mounted a transmission unit (11) for generating pulses of RF oscillations of a first frequency (f₁) and a transmission antenna (116) for emitting the pulses of RF oscillations, thereby exciting nuclear magnetization in the object to be examined in a near field of the transmission antenna in such a manner that said magnetization can be picked up by an RF receiving coil (5,6) of the MR imaging apparatus and reproduced in an MR image so as to visualize a position of the catheter.

2. A catheter as claimed in claim 1, **characterized in that** the transmission unit is formed by a microchip (11) which includes an oscillator (113) and is accommodated in a wall (10a) of the catheter.

3. A catheter as claimed in claim 2, **characterized in that** the microchip (11) includes at least one sensor (114, 114a) for the acquisition of measuring values of the object to be examined, as well as a modulator (115) for modulating RF oscillations of a second frequency (f₂), generated by the oscillator (113), with the measuring values of the sensor in such a manner that the measuring values can be transferred for evaluation to a receiving and evaluation unit (42) which is situated outside the object to be examined.

4. A catheter as claimed in claim 1, **characterized in that** the transmission unit (11) is connected, via a lead (111) which is guided through the catheter (10), to a power supply and/or control unit (12) which is situated outside the object to be examined.

5. A catheter as claimed in claim 1, **characterized in that** the transmission antenna is formed by at least one microcoil (116) which is wound around the catheter (10).

6. A catheter as claimed in claim 1, **characterized in that** the tip of the catheter is constructed as an expansion catheter (100, 101') and that the transmission antenna (116) is formed by turns of at least one microcoil which extend in walls (101a) of the expansion catheter.

7. A method of forming an MR image of an object to be examined by means of a magnetic resonance imaging apparatus as well as of visualizing in the MR image a position of a catheter as claimed in claim 1 which is introduced into the object to be examined, **characterized in that** switching over takes place between a first mode of operation and a second mode of operation in an alternating fashion, the RF transmission coil (4) of the MR imaging apparatus being operative so as to generate an MR image of the object to be examined in the first mode of operation while the transmission unit (11) of the catheter (10) is switched off, whereas in the second mode of operation the RF transmission coil (4) is switched off and the transmission unit (11) of the catheter is operative so as to excite nuclear magnetization in the near field of the transmission antenna (16), which nuclear magnetization is picked up by the MR imaging apparatus and reproduced in the MR image formed in the first mode of operation, thus visualizing the position of the catheter.

8. A method of forming an MR image of an object to be examined by means of a magnetic resonance imaging apparatus, as well as of visualizing in the MR image a position of a catheter as claimed in claim 1 which is introduced into the object to be examined, **characterized in that** the RF transmission coil (4) of the MR imaging apparatus and the transmission unit (11) of the catheter (10) are operative essentially simultaneously so that intensified nuclear magnetization is excited in the near field of the transmission antenna (16), which nuclear magnetization is picked up by the MR imaging apparatus and reproduced in the MR image formed, thus visualizing the position of the catheter.

9. A method as claimed in claim 7 or 8, **characterized in that** during operation of the transmission unit (11) of the catheter (10) switching over takes place in an alternating fashion between a first and a second frequency (f₁, f₂), the first frequency (f₁) being chosen to be such that the nuclear magnetization in the near field of the transmission antenna (116) is excited and the second frequency (f₂) serves to transfer measuring values of the object to be examined, as picked up by sensors (114, 114a), to a receiving and evaluation unit (42).

10. A method as claimed in claim 7 or 8, **characterized in that** during operation of the RF transmission coil (4) a voltage is induced in an inductance of the transmission unit (11), said voltage charging a capacitance which serves as a supply voltage source for the transmission unit (11) during operation of this unit.

11. A magnetic resonance imaging apparatus for carrying out the method claimed in claim 7 or 8, **characterized in that** it includes a switching unit (41) for manually and/or automatically activating the operation of the RF transmission coil (4) and the transmission unit (11), that is, in an alternating fashion or essentially simultaneously.

12. A magnetic resonance imaging apparatus for carrying out the method claimed in claim 9, **characterized in that** it includes a receiving and evaluation unit (42) provided with a receiving antenna array (7x, 8x) for receiving and evaluating the measuring values from the sensors (114, 114a) which are emitted with the second frequency (f₂) by the transmission unit 11.

## Patentansprüche

1. Katheter für die Verwendung während der Untersuchung eines Objekts mittels eines MR-Bildgebungsgeräts mit einer HF-Sendespule (4), wobei am Katheter (10) eine Sendeeinheit (11) zum Erzeugen hochfrequenter Schwingungsimpulse einer ersten Frequenz (f₁) und eine Sendeantenne (116) zum Emittieren der hochfrequenten Schwingungsimpulse angebracht sind, wodurch im Nahfeld der Sendeantenne eine Kernmagnetisierung im zu untersuchenden Objekt dergestalt angeregt wird, dass die genannte Magnetisierung von einer HF-Empfangsspule (5, 6) des MR-Bildgebungsgeräts aufgenommen und in einem MR-Bild wiedergegeben werden kann, um eine Position des Katheters sichtbar zu machen.

2. Katheter nach Anspruch 1, **dadurch gekennzeichnet, dass** die Sendeeinheit durch einen Mikrochip (11) gebildet wird, der einen Oszillator (113) enthält und in einer Wand (10a) des Katheters untergebracht ist.

3. Katheter nach Anspruch 2, **dadurch gekennzeichnet, dass** der Mikrochip (11) sowohl mindestens einen Sensor (114, 114a) zur Erfassung von Messwerten des zu untersuchenden Objekts als auch einen Modulator (115) zum Modulieren hochfrequenter Schwingungen einer zweiten vom Oszillator (113) erzeugten Frequenz (f₂) enthält, wobei die Messwerte des Sensors dergestalt sind, dass sie zur Auswertung an eine Empfangs- und Auswertungseinheit (42) übertragen werden können, die sich außerhalb des zu untersuchenden Objekts befindet.

4. Katheter nach Anspruch 1, **dadurch gekennzeichnet, dass** die Sendeeinheit (11) über eine Leitung (111), die durch den Katheter (10) geführt wird, mit einer Stromversorgungs- und/oder Steuereinheit (12) verbunden ist, die sich außerhalb des zu untersuchenden Objekts befindet.

5. Katheter nach Anspruch 1, **dadurch gekennzeichnet, dass** die Sendeantenne durch mindestens eine Mikrospule (116) gebildet wird, die um den Katheter (10) gewickelt ist.

6. Katheter nach Anspruch 1, **dadurch gekennzeichnet, dass** die Spitze des Katheters als ein Expansionskatheter (100, 101') ausgeführt ist und die Sendeantenne (116) durch Wicklungen mindestens einer Mikrospule gebildet wird, die sich in Wänden (101a) des Expansionskatheters erstrecken.

7. Verfahren sowohl zum Erzeugen eines MR-Bildes eines zu untersuchenden Objekts mittels eines Magnetresonanz-Bildgebungsgeräts als auch zum Visualisieren der Position eines in das zu untersuchende Objekt eingeführten Katheters (10) nach Anspruch 1 in dem MR-Bild, **gekennzeichnet dadurch, dass** das Umschalten zwischen einer ersten Betriebsart und einer zweiten Betriebsart in abwechselnder Weise stattfindet, wobei die HF-Sendespule (4) des MR-Bildgebungsgeräts arbeitet, um in der ersten Betriebsart ein MR-Bild des zu untersuchenden Objekts zu erzeugen, während die Sendeeinheit (11) des Katheters (10) ausgeschaltet ist, während in der zweiten Betriebsart die HF-Sendespule (4) ausgeschaltet ist und die Sendeeinheit (11) des Katheters arbeitet, um im Nahfeld der Sendeantenne (16) eine Kernmagnetisierung anzuregen, die vom MR-Bildgebungsgerät erfasst und in dem in der ersten Betriebsart erzeugten MR-Bild wiedergegeben wird, wodurch die Position des Katheters sichtbar gemacht wird.

8. Verfahren sowohl zum Erzeugen eines MR-Bildes eines zu untersuchenden Objekts mittels eines Magnetresonanz-Bildgebungsgeräts als auch zum Visualisieren der Position eines in das zu untersuchende Objekt eingeführten Katheters (10) nach Anspruch 1, **gekennzeichnet dadurch, dass** die HF-Sendespule (4) des MR-Bildgebungsgeräts und die Sendeeinheit (11) des Katheters (10) im Wesentlichen simultan arbeiten, so dass im Nahfeld der Sendeantenne (16) eine verstärkte Kernmagnetisierung angeregt wird, die vom MR-Bildgebungsgerät erfasst und im erzeugten MR-Bild wiedergegeben wird, wodurch die Position des Katheters sichtbar gemacht wird.

9. Verfahren nach Anspruch 7 oder 8, **dadurch gekennzeichnet, dass** während des Betriebs der Sendeeinheit (11) des Katheters (10) das Umschalten zwischen einer ersten und einer zweiten Frequenz (f₁, f₂) auf abwechselnde Weise stattfindet, wobei die erste Frequenz (f₁) so gewählt wird, dass die Kernmagnetisierung im Nahfeld der Sendeantenne (116) angeregt wird, und die zweite Frequenz (f₂) dazu dient, von Sensoren (114, 114a) erfassten Messwerte des zu untersuchenden Objekts an eine Empfangs- und Auswertungseinheit (42) zu übertragen.

10. Verfahren nach Anspruch 7 oder 8, **dadurch gekennzeichnet, dass** während des Betriebs der Sendespule (4) eine Spannung in eine Induktivität der Sendeeinheit (11) induziert wird, wobei die genannte Spannung einen Kondensator auflädt, der als Versorgungsspannungsquelle für die Sendeeinheit (11) während des Betriebs dieser Einheit dient.

11. Magnetresonanz-Bildgebungsgerät zum Ausführen des Verfahrens nach Anspruch 7 oder 8, **dadurch gekennzeichnet, dass** es eine Umschalteinheit (41) zum manuellen und/oder automatischen Aktivieren des Betriebs der HF-Sendespule (4) und der Sendeeinheit (11) beinhaltet, das heißt in einer abwechselnden oder im Wesentlichen simultanen Weise.

12. Magnetresonanz-Bildgebungsgerät zum Ausführen des Verfahrens nach Anspruch 9, **dadurch gekennzeichnet, dass** es eine Empfangs- und Auswertungseinheit (42) mit einem Empfangsantennenarray (7x, 8x) zum Empfangen und Auswerten der Messwerte von den Sensoren (114, 114a) beinhaltet, die von der Sendeeinheit (11) mit der zweiten Frequenz (f₂) ausgegeben werden.

## Revendications

1. Cathéter pour un usage pendant l'examen d'un objet au moyen d'un appareil d'imagerie RM avec une bobine de transmission RF (4), dans lequel sont montées sur le cathéter (10) une unité de transmission (11) pour générer des impulsions d'oscillations RF d'une première fréquence (f₁) et une antenne de transmission (116) pour émettre les impulsions d'oscillations RF de manière à activer une magnétisation nucléaire dans l'objet à examiner dans un champ proche de l'antenne de transmission de sorte que ladite magnétisation puisse être recueillie par une bobine réceptrice RF (5, 6) de l'appareil d'imagerie RM et reproduite dans une image RM de manière à visualiser une position du cathéter.

2. Cathéter selon la revendication 1, **caractérisé en ce que** l'unité de transmission est constituée d'une micropuce (11) qui comprend un oscillateur (113) et est aménagée dans une paroi (10a) du cathéter.

3. Cathéter selon la revendication 2, **caractérisé en ce que** la micropuce (11) comprend au moins un capteur (114, 114a) pour l'acquisition de valeurs de mesure de l'objet à examiner ainsi qu'un modulateur (115) pour moduler des oscillations RF d'une seconde fréquence (f₂), générées par l'oscillateur (113), avec les valeurs de mesure du capteur de sorte que les valeurs de mesure puissent être transférées pour être évaluées à une unité de réception et d'évaluation (42) qui est située à l'extérieur de l'objet à examiner.

4. Cathéter selon la revendication 1, **caractérisé en ce que** l'unité de transmission (11) est connectée, via un conducteur (111), qui est guidé à travers le cathéter (10), à une unité d'alimentation électrique et/ou de commande (12) qui est située à l'extérieur de l'objet à examiner.

5. Cathéter selon la revendication 1, **caractérisé en ce que** l'antenne de transmission est constituée d'au moins une microbobine (116) qui est enroulée autour du cathéter (10).

6. Cathéter selon la revendication 1, **caractérisé en ce que** la pointe des cathéters se présente sous la forme d'un cathéter à expansion (100, 101') et **en ce que** l'antenne de transmission (116) est constituée par des spires d'au moins une microbobine qui s'étendent dans les parois (101a) du cathéter à expansion.

7. Procédé pour former une image RM d'un objet à examiner au moyen d'un appareil d'imagerie à résonance magnétique ainsi que pour visualiser dans l'image RM une position d'un cathéter selon la revendication 1, qui est introduit dans l'objet à examiner, **caractérisé en ce qu'**une commutation se fait entre un premier mode opérationnel et un second mode opérationnel de manière alternée, la bobine de transmission RF (4) de l'appareil d'imagerie RM étant opérationnelle de manière à générer une image RM de l'objet à examiner dans le premier mode opérationnel alors que l'unité de transmission (11) du cathéter (10) est déconnectée, tandis que, dans le second mode opérationnel, la bobine de transmission (4) est déconnectée et l'unité de transmission (11) du cathéter est opérationnelle de manière à activer la magnétisation nucléaire dans le champ proche de l'antenne de transmission (16), laquelle magnétisation nucléaire est recueillie par l'appareil d'imagerie RM et reproduite dans l'image RM formée dans le premier mode opérationnel de manière à visualiser la position du cathéter.

8. Procédé pour former une image RM d'un objet à examiner au moyen d'un appareil d'imagerie à résonance magnétique ainsi que pour visualiser dans l'image RM une position d'un cathéter selon la revendication 1, qui est introduit dans l'objet à examiner, **caractérisé en ce que** la bobine de transmission RF (4) de l'appareil d'imagerie RM et l'unité de transmission (11) du cathéter (10) sont opérationnelles de manière sensiblement simultanée de sorte qu'une magnétisation nucléaire intensifiée soit activée dans le champ proche de l'antenne de transmission (16), laquelle magnétisation nucléaire est recueillie par l'appareil d'imagerie RM et reproduite dans l'image RM formée dans le premier mode opérationnel de manière à ainsi visualiser la position du cathéter.

9. Procédé selon la revendication 7 ou 8, **caractérisé en ce que**, durant le fonctionnement de l'unité de transmission (11) du cathéter (10), la commutation se fait de manière alternée entre une première et une seconde fréquence (f₁, f₂), la première fréquence (f₁) étant choisie de sorte que la magnétisation nucléaire dans le champ proche de l'antenne de transmission (116) soit activée et que la seconde fréquence (f₂) serve à transférer des valeurs de mesure de l'objet à examiner, telles que celles recueillies par des capteurs (114, 114a), à une unité de réception et d'évaluation (42).

10. Procédé selon la revendication 7 ou 8, **caractérisé en ce que**, durant le fonctionnement de la bobine de transmission RF (4), on induit une tension dans une induction de l'unité de transmission (11), ladite tension chargeant une capacité qui sert de source de tension d'alimentation pour l'unité de transmission (11) durant le fonctionnement de cette unité.

11. Appareil d'imagerie à résonance magnétique pour mettre en oeuvre le procédé selon la revendication 7 ou 8, **caractérisé en ce qu'**il comprend une unité de commutation (41) pour activer manuellement et/ou automatiquement le fonctionnement de la bobine de transmission RF (4) et de l'unité de transmission (11), c'est-à-dire de manière alternée ou de manière sensiblement simultanée.

12. Appareil d'imagerie à résonance magnétique pour mettre en oeuvre le procédé selon la revendication 9, **caractérisé en ce qu'**il comprend une unité de réception et d'évaluation (42) pourvue d'un réseau d'antennes réceptrices (7x, 8x) pour recevoir et évaluer les valeurs de mesure des capteurs (114, 114a) qui sont émises à la seconde fréquence (f₂) par l'unité de transmission 11.
